# FASCICULE DE BREVET EUROPEEN

(11) **EP 2 864 009 B1**
(45) Date de publication et mention de la délivrance du brevet: **09.08.2023**
(21) Numéro de dépôt: 13729776.8
(22) Date de dépôt: 20.06.2013
(51) Int. Cl.: A61L 9/12, B01B 1/00, A61L 2/20

(54) **DISPOSITIF D'ÉVAPORATION D'UN LIQUIDE ET PROCÉDÉ ASSOCIÉ**
VORRICHTUNG ZUM VERDAMPFEN EINER FLÜSSIGKEIT UND ZUGEHÖRIGES VERFAHREN
DEVICE FOR EVAPORATING A LIQUID AND ASSOCIATED METHOD

(30) Priorité: 25.06.2012 FR 1255999
(43) Date de publication de la demande: 29.04.2015
(73) Titulaire: Xeda International S.A., 13670 Saint Andiol (FR)
(72) Inventeur: SARDO, Alberto, F-13160 Chateaurenard (FR)
(74) Mandataire: Lavoix
(86) Numéro de dépôt international: PCT/EP2013/062924
(87) Numéro de publication internationale: WO 2014/001201

(56) Documents cités:
- WO-A1-2012/031365
- WO-A2-2006/103998
- WO-A2-2009/144465
- BE-A1- 773 391
- FR-A1- 2 791 910
- US-A- 282 474
- US-A- 3 092 096

## Description

La présente invention concerne en général des dispositifs permettant d'effectuer un traitement de stérilisation, notamment par une vapeur d'huiles essentielles ou par des produits synthétiques volatiles, destinés à des locaux ou à des matières végétales. Dans le cas de denrées alimentaires telles que les fruits et les légumes, le traitement vise à prolonger leur conservation. Dans le cas de locaux, le traitement vise à assainir les locaux.

La présente invention concerne en outre des dispositifs destinés à augmenter l'humidité de locaux ou de chambres de stockage de matières végétales.

WO 2009/144465 décrit que les vapeurs d'huiles essentielles, comme par exemple des huiles essentielles extraites d'agrumes, peuvent être utilisées pour traiter des aliments frais et détruire ou inhiber la croissance de microorganismes contaminant ces aliments.

Le traitement industriel de denrées alimentaires telles que des fruits et légumes stockés dans des chambres de stockage, des serres ou des silos nécessitent la production de quantités importantes de vapeurs. Par ailleurs, les vapeurs ne doivent pas condenser et former des gouttelettes d'huiles essentielles, de produits synthétiques volatiles ou d'eau qui puissent retomber sur les denrées alimentaires. De telles retombées peuvent en effet être phytotoxiques.

BE773391 A1 divulgue un dispositif d'évaporation d'un liquide, pour la désinfection de locaux.

US3092096 A divulgue un dispositif d'évaporation d'eau, pour une chaudière.

US282474 divulgue un appareil de ventilation et de refroidissement pour des bâtiments, avec un dispositif d'évaporation d'eau.

Dans ce contexte, il existe un besoin pour un dispositif permettant de produire des quantités importantes de vapeurs, sans risque de condensation.

A cette fin, la présente divulgation porte selon un premier aspect non couvert par les revendications sur un dispositif d'évaporation d'un liquide, le dispositif comportant :
- une réserve contenant le liquide ;
- une organe d'absorption du liquide ; et
- un organe de production d'un flux de gaz, le flux de gaz étant dirigé vers l'organe d'absorption ;
caractérisé en ce que l'organe d'absorption comprend une pluralité de bandes absorbantes propres à retenir le liquide.

Le fait que l'organe d'absorption comprenne une pluralité de bandes absorbantes permet d'obtenir une surface importante de contact entre le flux de gaz et l'organe d'absorption, et donc une surface importante d'évaporation du liquide. En outre, les bandes absorbantes sont avantageusement espacées les unes des autres de sorte que l'organe d'absorption est propre à fournir un espace important de passage du flux de gaz. Ainsi, le dispositif permet d'évaporer une quantité importante de liquide.

Typiquement, le liquide comprend au moins une huile essentielle. Par exemple, le liquide comporte une huile essentielle choisie dans le groupe formé par l'huile de menthe, l'huile de girofle, l'huile de rose, l'huile de thym, l'huile d'origan. En variante, le liquide comprend l'un des constituants de ces huiles choisi dans l'ensemble formé par le L-carvone, l'eugénol, le géraniol, le thymol, ou le carvacrol.

En variante, le liquide comprend au moins un produit de synthèse volatile ayant des propriétés biocides, tel que le diméthyl-naphtalène, le 3-décène-2-one ou l'hexanal.En variante, la composition liquide comporte du pyrèthre ou des pyréthrines de synthèse.

Typiquement, le liquide comporte seulement un produit actif sans solvant ni adjuvant.

Par produit actif on entend une huile essentielle ou un produit de synthèse volatile.

En variante, le liquide comporte un solvant aqueux ou organique, dans lequel est dissout le produit actif, et/ou un ou plusieurs adjuvants. Le solvant aqueux est par exemple de l'eau. Le solvant organique est par exemple un solvant du type décrit dans FR 2791910 ou des glycols, diglycols et leurs esters relatifs. Les adjuvants sont par exemple des substances aptes à véhiculer la ou les matières actives ou aptes à donner un effet de dilution.

En variante, le liquide comporte un mélange de plusieurs produits actifs liquides, par exemple plusieurs huiles essentielles de la liste ci-dessus, ou plusieurs produits de synthèses volatiles de la liste ci-dessus.

En variante, le liquide comporte uniquement de l'eau. Dans ce cas, la vaporisation de l'eau liquide par le dispositif d'évaporation est destinée à augmenter l'humidité du local ou de la chambre de stockage de matières végétales dans lequel ou laquelle le liquide est vaporisé.

Typiquement, le dispositif est propre à vaporiser le liquide à une température inférieure à 50°C, c'est-à-dire que le flux de gaz est à une température inférieure à 50°C, de préférence inférieure à 20°C, notamment comprise entre -2°C et 12°C et en particulier entre 0 et 10°C. Le flux de gaz est, par exemple, à température ambiante. Le dispositif permet donc une évaporation du liquide sans nécessiter de moyens de chauffage. Le dispositif ne nécessite pas non plus de buses d'injection du liquide à haute pression, qui impliquent généralement des coûts d'installation élevés, et qui ne permettent pas d'éviter la condensation ultérieure du liquide évaporé.

Typiquement, chaque bande est réalisée en microfibres végétales. En variante, chaque bande est réalisée en fibre synthétique. Chaque bande est, par exemple, formée par 80% de polyester et 20% de polyamide.

Suivant un premier mode de réalisation, le dispositif comprend un organe d'injection du liquide dans l'organe d'absorption à partir de la réserve. On entend ici par injection le fait d'introduire par une action volontaire, positive, une quantité de liquide dans l'organe d'absorption.

De préférence, l'organe d'injection comprend une pompe doseuse. Un tel organe doseur permet de contrôler de manière précise la quantité de liquide injecté. En variante, le liquide est injecté par gravité, par effet venturi, ou par tout autre organe doseur adapté.

Typiquement, la pompe doseuse et l'organe de production du flux de gaz sont contrôlés par un calculateur. En variante, la pompe doseuse et/ou l'organe de production du flux de gaz sont contrôlés manuellement.

Selon ce premier mode de réalisation, chaque bande présente une première extrémité et une seconde extrémité et l'organe d'injection présente une sortie d'éjection du liquide, la sortie d'éjection étant disposée à proximité de la première extrémité de chaque bande. Ainsi, le liquide est injecté dans la première extrémité de chaque bande et s'écoule par gravité le long de chaque bande absorbante vers la seconde extrémité.

Suivant ce premier mode de réalisation, la première extrémité de chaque bande est typiquement située au pôle d'une sphère, chaque bande s'étendant depuis le pôle suivant une longitude de ladite sphère. Cette configuration permet d'optimiser l'évaporation du liquide retenu dans les bandes absorbantes. En variante, l'organe d'absorption présente une forme en cône, en patatoïde ou toute forme concave.

Suivant ce premier mode de réalisation, l'organe de production de flux de gaz est dirigé vers le pôle, du côté concave de la sphère. Cette disposition de l'organe de production de flux de gaz par rapport à l'organe d'absorption permet au flux de gaz d'être dirigé de façon à optimiser l'évaporation du liquide. Elle permet en effet une meilleure répartition du flux d'air par rapport aux bandes absorbantes qui retiennent le liquide destiné à être évaporé.

L'organe de production de flux de gaz est, par exemple, un ventilateur.

Selon un second mode de réalisation, les bandes absorbantes sont disposées parallèlement les unes aux autres et s'étendent suivant un axe longitudinal, l'axe longitudinal étant perpendiculaire à la direction du flux de gaz. En variante, l'axe longitudinal est incliné par rapport à la direction du flux de gaz.

Selon une variante du second mode de réalisation non protégé par la revendication 1, le dispositif comprend un organe de stockage du liquide relié à la réserve, la seconde extrémité de chaque bande trempant dans le liquide de l'organe de stockage de façon à ce que chaque bande absorbe le liquide par capillarité.

Suivant ce second mode de réalisation, le dispositif comprend une conduite d'injection depuis la réserve vers l'organe de stockage du liquide. Typiquement et à l'instar de l'organe d'injection du premier mode de réalisation, la conduite d'injection comprend une pompe doseuse.

Selon une autre variante de ce second mode de réalisation et à l'instar du premier mode de réalisation, le dispositif comprend un organe d'injection du liquide dans l'organe d'absorption à partir de la réserve.

Selon cette variante, chaque bande présente une première extrémité et une seconde extrémité et l'organe d'injection présente une sortie d'éjection du liquide, la sortie d'éjection étant disposée à proximité de la première extrémité de chaque bande. Ainsi, le liquide est injecté dans la première extrémité de chaque bande absorbante verticale et s'écoule par gravité le long de chaque bande absorbante verticale.

Selon un second aspect non couvert par la revendication 1, la présente divulgation porte sur un ensemble de traitement d'un local, l'ensemble de traitement comprenant :
- un local ; et
- un dispositif d'évaporation ayant les caractéristiques ci-dessus..

L'invention porte sur un procédé d'évaporation d'un liquide selon la revendication 1.

D'autres caractéristiques et avantages de l'invention ressortiront de la description détaillée qui en est donnée ci-dessous, à titre indicatif et nullement limitatif, en référence aux figures annexées, parmi lesquelles :
- la figure 1 est une représentation schématique simplifiée d'un ensemble de traitement d'un local comprenant un premier mode de réalisation d'un dispositif d'évaporation d'un liquide; et
- la figure 2 est une représentation schématique simplifiée d'un ensemble de traitement d'un local comprenant un second mode de réalisation d'un dispositif d'évaporation d'un liquide.

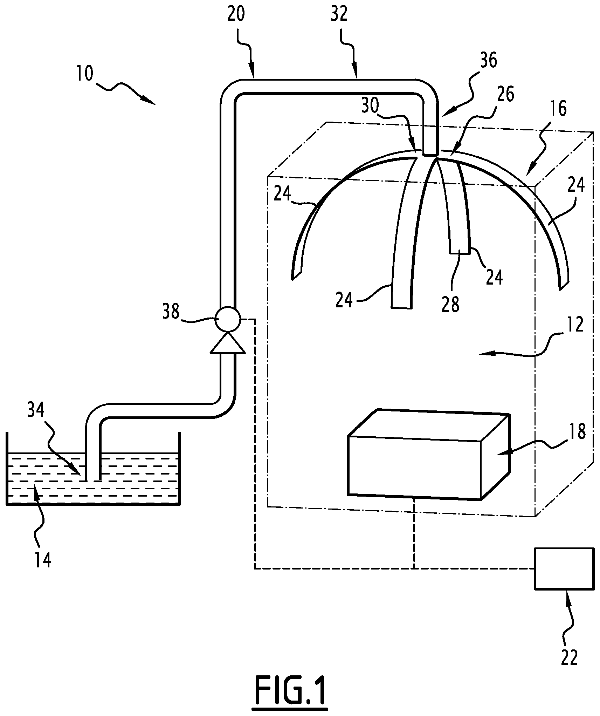

Sur la figure 1 est représenté un dispositif 10 d'évaporation d'un liquide selon un premier mode de réalisation.

Un tel dispositif 10 est typiquement prévu pour la stérilisation d'un local 12 ou d'une chambre de stockage de matières végétales telle qu'une serre dans le cas où le liquide à évaporer contient au moins une huile essentielle. Dans le cas où le liquide destiné à être évaporé comprend uniquement de l'eau, un tel dispositif 10 est prévu pour augmenter l'humidité du local 12 ou de la chambre de stockage.

Le dispositif 10 représenté sur la figure 1 comprend :
- une réserve 14 contenant le liquide ;
- un organe d'absorption 16 du liquide ;
- un organe de production 18 d'un flux de gaz ;
- un organe d'injection 20 du liquide propre à injecter le liquide dans l'organe d'absorption 16 à partir de la réserve 14 ; et
- un calculateur 22 pilotant l'organe d'injection 20 et l'organe de production 18 du flux de gaz.

L'organe d'absorption 16 du liquide comprend une pluralité de bandes absorbantes 24 propres à retenir le liquide. Par exemple, l'organe d'absorption présente entre 2 et 40 bandes absorbantes, notamment entre 4 et 20 bandes et en particulier entre 6 et 10 bandes.

Les bandes absorbantes 24 de l'organe d'absorption 16 sont par exemple réalisées en microfibres végétales. En variante, les bandes absorbantes 24 sont réalisées en fibres synthétiques.

Suivant le premier mode de réalisation représenté sur la figure 1, chaque bande absorbante 24 présente une première extrémité 26 et une seconde extrémité 28. La première extrémité 26 de chaque bande absorbante 24 est, avantageusement, située au pôle 30 d'une sphère. Chaque bande absorbante 24 s'étend depuis le pôle 30 suivant une longitude de la sphère.

Typiquement, les bandes absorbantes 24 sont mises en forme par l'intermédiaire de la pression du flux de gaz exercée sur les bandes absorbantes 24.

L'organe de production 18 de flux de gaz est, de manière avantageuse, dirigé vers le pôle 30 de la sphère. L'organe de production 18 de flux de gaz est disposé du côté concave de la sphère, en contrebas de cette dernière.

En variante, l'organe de production 18 de flux de gaz est placé en amont de l'organe d'absorption 16. Le terme « amont » s'entend ici selon l'écoulement normal du liquide dans le dispositif 10.

L'organe de production 18 de flux de gaz est, par exemple, un ventilateur 18.

L'organe d'injection 20 présente un conduit d'injection 32 du liquide. Le conduit d'injection 32 est par exemple un conduit ouvert à ses deux extrémités. L'une des extrémités définit une entrée 34 de liquide et est raccordée à la réserve 14. L'extrémité opposée définit une sortie d'éjection 36 du conduit d'injection 32. La sortie d'éjection 36 est disposée à proximité de la première extrémité 26 de chaque bande absorbante 24. L'organe d'injection 20 comprend une pompe doseuse 38.

Le fonctionnement du dispositif 10 selon le premier mode de réalisation va maintenant être décrit.

Initialement, un utilisateur entre dans le calculateur 22 des valeurs de consigne pour le débit de liquide à injecter par la pompe doseuse 38 et pour le temps de pause (le ventilateur tourne toujours et à la même vitesse). Le liquide est initialement stocké dans la réserve 14.

Puis, l'organe d'injection 20 du liquide pompe, par l'intermédiaire de la pompe doseuse 38, le liquide depuis la réserve 14 à travers le conduit d'injection 32 jusqu'à la sortie d'éjection 36.

Ensuite, le liquide sort du conduit d'injection 32 par la sortie d'éjection 36 puis est absorbé par les bandes absorbantes 24 de l'organe d'absorption 16. Le liquide est injecté dans la première extrémité 26 de chaque bande absorbante 24 puis s'écoule le long de chaque bande absorbante 24 jusqu'à la seconde extrémité 28 de chaque bande absorbante 24.

Simultanément, le flux d'air est produit, par l'intermédiaire du ventilateur 18. Le flux d'air est dirigé vers le pôle 30 de la sphère que forment les bandes absorbantes 24. Le ventilateur 18 est avantageusement disposé du côté concave de la sphère, à l'intérieur de la sphère ou en contrebas de celle-ci.

Enfin, le liquide retenu dans les bandes absorbantes 24 de l'organe d'absorption 16 est évaporé par l'intermédiaire du flux d'air dirigé vers les bandes absorbantes 24.

Les bandes absorbantes 24 de l'organe d'absorption 16 permettent de fournir une surface d'évaporation importante, et leur disposition les unes par rapport aux autres permet un passage facilité du flux d'air et donc une évaporation importante du liquide retenu dans les bandes absorbantes 24.

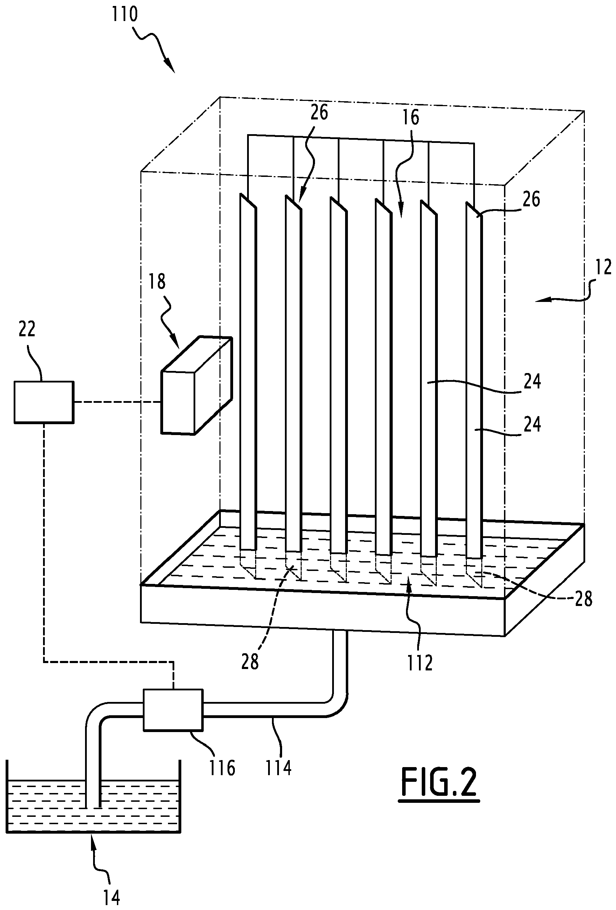

Un second mode de réalisation 110 du dispositif d'évaporation d'un liquide est représenté schématiquement sur la figure 2.

A la différence du premier mode de réalisation représenté sur la figure 1, les bandes absorbantes 24 sont disposées parallèlement les unes aux autres et s'étendent suivant un axe longitudinal vertical.

En outre, le dispositif 110 selon le second mode de réalisation comprend un organe de stockage du liquide relié à la réserve 14. L'organe de stockage 112 est par exemple un bac disposé dans le local 12 et dans lequel est stocké le liquide. L'organe de stockage 112 est relié à la réserve 14 par l'intermédiaire d'une conduite d'injection 114 et comprend avantageusement une pompe doseuse 116 reliée au calculateur 22.

L'organe de stockage 112 est propre à stocker le liquide à l'intérieur du local 12 et est avantageusement disposé en contrebas des bandes absorbantes 24.

Selon ce second mode de réalisation, la seconde extrémité 28 de chaque bande absorbante 24 trempe dans le liquide présent dans le bac 112. Chaque bande absorbante 24 absorbe ainsi le liquide par capillarité.

Dans ce second mode de réalisation, le ventilateur 18 est avantageusement disposé de façon à produire un flux d'air horizontal dirigé vers les bandes absorbantes 24 verticales. Ainsi, le flux d'air est perpendiculaire au plan dans lequel sont contenues les bandes absorbantes 24.

En variante, le dispositif 110 selon le second mode de réalisation comprend l'organe d'injection 20 précédemment décrit. Dans cette variante (non représentée), à l'instar du fonctionnement du dispositif 110 selon le premier mode de réalisation, le liquide est pompé depuis la réserve 14 jusqu'à la sortie d'éjection 36 de l'organe d'injection 20 par une pompe doseuse. De plus, la sortie d'éjection 36 comprend une pluralité de canaux qui relient le conduit d'injection 32 à la première extrémité 26 de chaque bande absorbante.

Le liquide est ensuite injecté dans la première extrémité 26 de chaque bande absorbante 24 puis s'écoule verticalement le long de chaque bande absorbante 24. Le liquide est ensuite évaporé par l'intermédiaire du flux de gaz horizontal dirigé vers les bandes absorbantes 24 verticales.

On conçoit ainsi que le dispositif, et le procédé d'évaporation d'un liquide selon l'invention, permettent de produire des concentrations élevées d'huile essentielle, éventuellement jusqu'à légèrement en dessous de la saturation de l'huile essentielle dans l'atmosphère, et ce pour des périodes longues et pour des volumes importants.

Le risque de condensation de l'huile essentielle sur les denrées stockées, et donc la phytotoxicité de l'huile essentielle, sont donc évités, car à l'approche de la concentration de saturation dans l'atmosphère, l'évaporation n'aura plus lieux et il n'y a pas le risque de sursaturation.

## Revendications

1. Procédé d'évaporation d'un liquide comprenant les étapes suivantes :
- absorption du liquide par un organe d'absorption (16) comprenant une pluralité de bandes absorbantes (24) propres à retenir le liquide, chaque bande présentant une première extrémité (26) et une seconde extrémité (28), le liquide comprenant au moins une huile essentielle, un produit synthétique volatile ou un produit de synthèse volatile ayant des propriétés biocides ;
- production d'un flux de gaz, le flux de gaz étant dirigé vers l'organe d'absorption (16) ;
**caractérisé en ce que** la méthode comprend une étape d'évaporation du liquide à une température inférieure à 50°C, le flux de gaz étant à une température inférieure à 50°C ;
**en ce que** le liquide est injecté dans l'organe d'absorption (16) à partir du réservoir (14) par un organe d'injection (20), l'organe d'injection (20) comprenant une pompe doseuse (38),
et **en ce que** l'organe d'injection (20) présente une sortie d'éjection (36) du liquide, la sortie d'éjection (36) étant disposée à proximité de la première extrémité (26) de chaque bande, de telle sorte que le liquide est injecté dans la première extrémité (26) de chaque bande (24) et s'écoule par gravité le long de chaque bande (24) absorbante vers la seconde extrémité (28).

2. Procédé d'évaporation selon la revendication 1, **caractérisé en ce que** la ou chaque huile essentielle est choisie dans le groupe formé par l'huile de menthe, l'huile de girofle, l'huile de rose, l'huile de thym, l'huile d'origan ou au moins l'un de leurs constituants choisi dans l'ensemble formé par le L-carvone, l'eugénol, le géraniol, le thymol, le carvacrol, le pyrèthre, les pyréthrines synthétiques.

3. Procédé d'évaporation selon la revendication 1 ou 2, **caractérisé en ce que** la première extrémité (26) de chaque bande est située au pôle (30) d'une sphère, chaque bande s'étendant depuis le pôle (30) suivant une longitude de ladite sphère.

4. Procédé d'évaporation selon la revendication 3, **caractérisé en ce que** le flux de gaz est dirigé vers le pôle (30) du côté concave de la sphère.

5. Procédé d'évaporation selon l'une quelconque des revendications précédentes, **caractérisé en ce que** chaque bande est réalisée en microfibres végétales.

## Patentansprüche

1. Verfahren zum Verdampfen einer Flüssigkeit, umfassend die folgenden Schritte:
- Absorbieren der Flüssigkeit durch ein Absorptionselement (16), umfassend eine Vielzahl von absorbierenden Streifen (24), die geeignet sind, um die Flüssigkeit zurückzuhalten, wobei jeder Streifen ein erstes Ende (26) und ein zweites Ende (28) aufweist, die Flüssigkeit umfassend mindestens ein ätherisches Öl, ein flüchtiges synthetisches Produkt oder ein flüchtiges synthetisches Produkt, das biozide Eigenschaften aufweist;
- Erzeugen eines Gasstroms, wobei der Gasstrom auf das Absorptionselement (16) gerichtet wird;
**dadurch gekennzeichnet, dass** das Verfahren einen Schritt eines Verdampfens der Flüssigkeit bei einer Temperatur von weniger als 50 °C umfasst, wobei der Gasstrom eine Temperatur von weniger als 50 °C aufweist;
dass die Flüssigkeit aus dem Behälter (14) durch ein Einspritzelement (20) in das Absorptionselement (16) eingespritzt wird, das Einspritzelement (20) umfassend eine Dosierpumpe (38),
und dass das Einspritzorgan (20) einen Ausstoßausgang (36) der Flüssigkeit aufweist, wobei der Ausstoßausgang (36) in der Nähe des ersten Endes (26) von jedem Streifen angeordnet ist, sodass die Flüssigkeit in das erste Ende (26) von jedem Streifen (24) eingespritzt wird und durch Schwerkraft entlang jedes absorbierenden Streifens (24) zu dem zweiten Ende (28) fließt.

2. Verdampfungsverfahren nach Anspruch 1, **dadurch gekennzeichnet, dass** das oder jedes ätherische Öl ausgewählt ist aus der Gruppe, gebildet von Minzöl, Nelkenöl, Rosenöl, Thymianöl, Oreganoöl oder mindestens einem ihrer Bestandteile, ausgewählt aus der Gruppe, gebildet von L-Carvon, Eugenol, Geraniol, Thymol, Carvacrol, Pyrethrum, synthetischen Pyrethrinen.

3. Verdampfungsverfahren nach Anspruch 1 oder 2, **dadurch gekennzeichnet, dass** sich das erste Ende (26) von jedem Streifen an dem Pol (30) einer Kugel befindet, wobei sich jeder Streifen von dem Pol (30) entlang einer Länge der Kugel erstreckt.

4. Verdampfungsverfahren nach Anspruch 3, **dadurch gekennzeichnet, dass** der Gasstrom auf den Pol (30) der konkaven Seite der Kugel gerichtet ist.

5. Verfahren nach einem der vorherigen Ansprüche, **dadurch gekennzeichnet, dass** jeder Streifen aus pflanzlichen Mikrofasern gefertigt ist.

## Claims

1. Method for evaporating a liquid comprising the following steps:
- absorption of the liquid by an absorption organ (16) comprising a plurality of absorbent strips (24) suited to retain the liquid, each strip having a first end (26) and a second end (28), the liquid comprising at least one essential oil, a volatile synthetic product, or a volatile synthetic product with biocidal properties;
- production of a gas flow, which is directed towards the absorption organ (16);
**characterized in that** the method comprises a step of evaporation of the liquid at a temperature below 50 °C, the gas flow being at a temperature below 50 °C; **in that** the liquid is injected into the absorption organ (16) from the reservoir (14) by an organ for injecting (20) comprising a dosing pump (38),
and **in that** the injection organ (20) has a liquid injection output (36), liquid injection output (36) being arranged near the first end (26) of each strip, such that the liquid is injected into the first end (26) of each strip (24) and flows by gravity along each absorbent strip (24) towards the second end (28).

2. Evaporation method according to claim 1, **characterised in that** the/each essential oil is chosen from mint oil, clove oil, rose oil, thyme oil, oregano oil, or at least one of their constituents from the group of L-carvone, eugenol, geraniol, thymol, carvacrol, pyrethrum or synthetic pyrethrins.

3. Evaporation method according to claim 1 or 2, **characterised in that** the first end (26) of each strip is arranged at the pole (30) of a sphere, with each strip extending from the pole (30) along a longitude of the sphere.

4. Evaporation method according to claim 3, **characterised in that** the gas flow is aimed at the pole (30) on the concave side of the sphere.

5. Evaporation method according to any of the foregoing claims, **characterised in that** each strip consists of plant microfibres.
